# EUROPÄISCHE PATENTSCHRIFT

(11) **EP 0 993 828 B1**
(45) Veröffentlichungstag und Bekanntmachung des Hinweises auf die Patenterteilung: **03.07.2002**
(21) Anmeldenummer: 99117277.6
(22) Anmeldetag: 02.09.1999
(51) Int. Cl.: A61K 9/28, A61K 9/20

(54) **Verfahren zur Herstellung von beschichteten festen Dosierungsformen**
Method of producing coated solid dosage forms
Procédé pour produire des formes de dosage solides enrobées

(30) Priorität: 03.09.1998 DE 19840256
(43) Veröffentlichungstag der Anmeldung: 19.04.2000
(73) Patentinhaber: BASF AKTIENGESELLSCHAFT, 67056 Ludwigshafen (DE)
(72) Erfinder: Breitenbach, Jörg, 68199 Mannheim (DE); Kothrade, Stephan, 67117 Limburgerhof (DE); Kleinke, Andreas, 69121 Heidelberg (DE); Lange, Armin, 69121 Heidelberg (DE); Maier, Werner, 67105 Schifferstadt (DE)
(74) Vertreter: Kinzebach, Werner, Dr.

(56) Entgegenhaltungen:
- WO-A-89/09066
- WO-A-96/19963
- WO-A-97/15293
- GB-A- 2 249 957

## Beschreibung

Die vorliegende Erfindung betrifft ein Verfahren zur Herstellung von beschichteten festen Dosierungsformen durch Bilden eines plastischen Gemischs, enthaltend mindestens ein polymeres Bindemittel und mindestens einen Wirkstoff, und Extrudieren des plastischen Gemischs.

Beschichtete feste Dosierungsformen finden, insbesondere für pharmazeutische Zwecke, immer häufiger Anwendung. Beispiele für beschichtete feste Dosierungsformen sind Dragees, Filmtabletten, Manteltabletten, Mehrschichtfilmtabletten und Mehrschichtdragees. Dragees und Filmtabletten erhält man durch Behandeln (Coating) eines wirkstoffhaltigen Bindemittels (z. B. Granulat, Tablette) mit geeigneten filmbildenden Substanzen, die den Geschmack der Wirkstoffe überdecken, die Feuchtigkeits- und Lagerstabilität erhöhen und die Identifizierung erleichtern sollen. Durch geeignete Auswahl der Überzüge kann jedoch auch die Charakteristik der Wirkstofffreisetzung beeinflusst werden. Bei Manteltabletten, Mehrschichtfilmtabletten oder Mehrschichtdragees werden mehrere Schichten oder Lagen Bindemittel, in der Regel in aufwendigen, meist mehrstufigen Verfahren, in Tablettiermaschinen miteinander verpresst. Die unterschiedlichen Schichten enthalten meist unterschiedliche Wirkstoffe oder Wirkstoffe in unterschiedlichen Konzentrationen, um die gemeinsame Applikation unverträglicher Wirkstoffe zu ermöglichen und/oder die Freisetzungscharakteristik gezielt zu beeinflussen. Die Verfahren zur Herstellung solcher festen Dosierungsformen sind im Allgemeinen aufwendig, mehrstufig und deshalb zeit- und kostenintensiv.

Seit einiger Zeit ist ein wesentlich einfacheres kontinuierliches Verfahren zur Herstellung fester Arzneiformen bekannt, bei dem man eine wirkstoffhaltige lösungsmittelfreie Schmelze aus einem polymeren wirkstoffhaltigen Bindemittel extrudiert und den extrudierten Strang zu der gewünschten Arzneiform, z. B in einem Kalander mit Formwalzen, formt, siehe beispielsweise EP-A-0 240 904 und EP-A-0 240 906. Mit diesem Verfahren lassen sich feste Kombinationsarzneiformen, wie in der WO 97/15293 beschrieben, herstellen. Die Herstellung von mehrschichtigen festen Arzneiformen mit diesem Verfahren gelingt durch Coextrusion, wie in der DE 197 10 213.1 beschrieben, jedoch ist dieses Verfahren zur Verarbeitung der für die Herstellung hochretardierender Dosierungsformen wichtigen hochzähen Bindemittel für innenliegende Phasen oder Schichten nur bedingt geeignet,da diese leicht zur Verstopfung der formgebenden Düsen führen können.

Die WO 96/19963 beschreibt ein Verfahren zur Herstellung von umhüllten Tabletten durch Schmelzkalandrierung, wobei die wirkstoffhaltige Schmelze zwischen zwei Folien aus dem Umhüllungsmaterial in die Kalanderformwalzen eingeführt wird. Dieses Verfahren ist jedoch auf thermoplastische Überzugsmassen beschränkt, die sich als Folien verarbeiten lassen.

Die WO-A-89/09066 offenbart ein Verfahren, wobei PEG 35.000 und PEG 400 Monostearat zusammen geschmolzen werden einen Wirkstoff zugesetzt wird, und dieses Gemisch in ein Teflon-Röhrchen extrudiert wird. Nach Abkühlung wird das Extrudat aus dem Rohr gedrückt und mit einer Polyurethanlösung überzogen. Das überzogene Extrudat wird in Segmente zerteilt.

Der vorliegenden Erfindung liegt daher die Aufgabe zugrunde, ein kontinuierliches Verfahren zur Herstellung beschichteter, fester Dosierungsformen bereitzustellen, das auch die Verwendung von hochzähen Bindemitteln für innenliegende Schichten und die Verwendung einer Vielzahl von Beschichtungsmitteln erlaubt.

Es wurde nun überraschend gefunden, dass die Aufgabe gelöst wird, wenn man aus mindestens einem thermoplastischen polymeren Bindemittel und mindestens einem Wirkstoff ein plastisches Gemisch bildet und extrudiert, das Extrudat in einem zweiten Schritt mit mindestens einem flüssigen oder dampfförmigen Beschichtungsmittel behandelt und anschließend die Formgebung durchführt.

Gegenstand der Erfindung ist daher ein Verfahren zur Herstellung beschichteter fester Dosierungsformen durch Bilden eines plastischen Gemischs aus mindestens einem thermoplastischen, physiologisch verträglichen, polymeren Bindemittel und mindestens einem Wirkstoff und Extrudieren des plastischen Gemischs, wobei man anschließend das Extrudat mit mindestens einem flüssigen oder dampfförmigen Beschichtungsmittel behandelt und das beschichtete Extrudat zur gewünschten Dosierungsform formt, das dadurch gekennzeichnet ist, dass man das mit dem Beschichtungsmittel überzogene Extrudat zur Einstellung der Beschichtungsdicke und der Beschichtungsform durch eine kalibrierte Düse führt.

Für die Herstellung des plastischen Gemischs ist es erforderlich, die Bestandteile, nämlich mindestens ein thermoplastisches, physiologisch verträgliches, polymeres Bindemittel und mindestens einen Wirkstoff und gegebenenfalls übliche Additive zu vermischen und in ein plastisches Gemisch überzuführen, vorzugsweise in Abwesenheit eines Lösungsmittels. Die Bildung des plastischen Gemischs kann durch Aufschmelzen oder auch durch Kneten, Vermischen oder Homogenisieren unterhalb der Schmelztemperatur des Bindemittels durchgeführt werden. Diese Verfahrensschritte können auf bekannte Art und Weise durchgeführt werden, beispielsweise wie in der EP-A-0 240 904, EP-A-0 337 256, EP-A-0 358 105, WO 97/15290 und WO 97/15291 beschrieben.

Die Komponenten können zuerst vermischt und dann in den plastischen Zustand überführt und homogenisiert werden. Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, in den plastischen Zustand überzuführen und vorzuvermischen, wobei die Apparaturen, wie Rührkessel, Rührwerke, Feststoffmischer etc., gegebenenfalls im Wechsel betrieben werden und dann den (die) empfindlichen Wirkstoff(e) in "Intensivmischern" in plastischer Phase bei sehr kleinen Verweilzeiten einzumischen (homogenisieren). Der (die) Wirkstoff(e) kann (können) in fester Form oder als Lösung oder Dispersion eingesetzt werden.

Das Aufschmelzen und Vermischen erfolgt in einer für diesen Zweck üblichen Vorrichtung. Besonders geeignet sind Extruder oder beheizbare Behälter mit Rührwerk, z. B. Kneter, (wie der unten noch erwähnten Art).

Als Mischapparat sind auch solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/ - Stator-Systeme (z. B. Dispax der Firma IKA).

Bei empfindlichen Wirkstoffen erfolgt vorzugsweise zunächst das Überführen des polymeren Bindemittels in den plastischen Zustand in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drükken von Vorteil ist, zugespeist werden. Flüssige Medien können über ein geeignetes Pumpenaggregat zudosiert werden.

Das durch Vermischen und Überführen des Bindemittels, gegebenenfalls des Wirkstoffes und gegebenenfalls des Additivs oder der Additive, in den plastischen Zustand erhaltene Gemisch ist teigig bis zähflüssig (thermoplastisch) und daher auch extrudierbar. Das Bindemittel soll vorzugsweise in physiologischer Umgebung löslich oder quellbar sein. Beispiele für geeignete Bindemittel sind:

Polyvinyllactame, insbesondere Polyvinylpyrrolidon (PVP), Copolymere von Vinyllactamen, wie N-Vinylpyrrolidon, N-Vinylpiperidon und N-Vinyl-ε-caprolactam, aber insbesondere N-Vinylpyrrolidon, mit (Meth)acrylsäure, (Meth)acrylsäureestern, Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate, Copolymerisate von Dimethylaminoethylacrylaten und Methacrylestern (z. B. EudragitR-Typen), Polyalkylenglykole, wie Polypropylenglykole und Polyethylenglykole (z.B. Polyethylenglykol 6000), Copolymerisate von Methylmethacrylat und Acrylsäure, Celluloseester, Celluloseether, insbesondere Methylcellulose und Ethylcellulose, Hydroxyalkylcellulosen, insbesondere Hydroxypropylcellulose oder Hydroxypropylmethylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane.

Brauchbar sind auch Gelatine und bioabbaubare Polymere, wie Polyhydroxyalkanoate, z. B. Polyhydroxybuttersäure, Polymilchsäure, Polyaminosäuren, z. B. Polylysin, Polyasparagin, Polydioxane u. Polypeptide.

Bevorzugte polymere Bindemittel sind Polyvinylpyrrolidon, Copolymerisate von N-Vinyllactamen, insbesondere N-Vinylpyrrolidon, und Vinylestern, Copolymerisate von N-Vinyllactamen, insbesondere N-Vinylpyrrolidon, mit (Meth)acrylsäureestern, Polyhydroxyalkylacrylate, Polyhydroxyalkylmethacrylate, Polyacrylate, Polymethacrylate, Alkylcellulosen und Hydroxyalkylcellulosen.

Vorteilhaft am erfindungsgemäßen Verfahren ist, dass es sich für Bindemittel sehr unterschiedlicher Viskosität eignet, beispielsweise für Bindemittel mit K-Werten (nach H. Fikentscher, Cellulose-Chemie 13 (1932), S. 58-64 und 71-74) zwischen 10 und 100, insbesondere zwischen 20 und 100. Insbesondere kommen die Vorteile dieses Verfahrens zum Tragen bei Bindemitteln, die einen K-Wert von > 45 und bevorzugt von > 50 aufweisen.

Das polymere Bindemittel muss sich in der Gesamtmischung aller Komponenten im Bereich von 50 bis 180 °C, vorzugsweise 60 bis 130 °C in einen plastischen Zustand überführen lassen. Die Glasübergangstemperatur der Mischung muss daher unter 200 °C, vorzugsweise unter 150 °C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-% bezogen auf das Polymerisat, betragen kann, sind z. B. Streckmittel bzw. Füllstoffe, wie Silikate oder Kieselerde, Magnesiumoxid, Aluminiumoxid, Titanoxid, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches;
Schmiermittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 50 °C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Schmier- und Trennmitteln beträgt vorzugsweise 0,1 bis 5 Gew.-%, bezogen auf das Gesamtgewicht der Masse für die jeweilige Schicht;
Fließmittel, z.B, AerosilR, in einer Menge von 0,1 bis 5 Gew.-%, bezogen auf das Gesamtgewicht des Gemisches;
Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al. Pharmazeutische Technologie, Thieme-Verlag, Stuttgart 1978).

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als pharmazeutische Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht wesentlich zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,001 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe, sowie Pflanzenbehandlungsmittel und Insektizide. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika und Impfstoffe.

Das erfindungsgemäße Verfahren ist beispielsweise zur Verarbeitung folgender Wirkstoffe bzw. der pharmakologisch aktiven Salze davon geeignet:
Acebutolol, Acetylcystein, Acetylsalicylsäure, Acyclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin, Captopril, Omeprazol, Ranitidin, Tramadol, Cyclosporin, Trandolapril und Peptidtherapeutika.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Das erfindungsgemäße Verfahren eignet sich zum einen zur Herstellung von beschichteten Dosierungsformen, die nur eine, in der Regel wirkstofffreie Beschichtung oder Umhüllung aufweisen, wie z. B. Dragees oder Filmtabletten. Zum anderen ermöglicht das erfindungsgemäße Verfahren eine große Variationsbreite bei der Herstellung von mehrschichtigen festen Dosierungsformen durch einfaches oder mehrfaches Behandeln des zuvor gebildeten Extrudats mit wirkstoffhaltigen und/oder wirkstofffreien Überzugsmassen, wodurch sich praktisch beliebige Wirkstoffkombinationen und Wirkstofffreisetzungscharakteristiken einstellen lassen.

Bei der Extrusion des plastischen Gemisches werden Temperatur, Viskosität und Extrusionsgeschwindigkeit vorteilhaft so gewählt, dass ein zusammenhängendes, selbstragendes Extrudat erhalten wird. In der Regel wird so kontinuierlich ein Strangextrudat mit vorzugsweise konstantem Querschnitt erzeugt. In Abhängigkeit von der Anzahl und Verträglichkeit der einzusetzenden Wirkstoffe können vorteilhaft auch mehrschichtige Extrudate, z. B. Coextrudate, wie in der WO 96/19963 beschrieben, bei dem erfindungsgemäßen Verfahren eingesetzt und mit mindestens einem Beschichtungsmittel ein- oder gegebenenfalls auch mehrmals behandelt werden, wobei das oder die Bindemittel gegebenenfalls einen oder mehrere Wirkstoffe enthalten können.

In der Regel werden Extrudate in Form eines Bandes oder eines Stranges, vorzugsweise mit rundem, ovalem oder abgerundetem Querschnitt, erhalten. In speziellen Ausführungsformen des erfindungsgemäßen Verfahrens können auch zwei oder mehrere Extrudate, etwa parallele Stränge oder Bänder oder, insbesondere bei Coextrudaten, Doppel- oder Mehrlingsstränge gemeinsam mit einem oder mehreren Beschichtungsmitteln behandelt werden.

Vorteilhafterweise ist bei dem erfindungsgemäßen Verfahren der Abstand zwischen Austritt des Extrudats und der Aufbringung des Überzuges in weiten Grenzen variabel. Durch Variation dieses Abstandes lässt sich, gegebenenfalls unter Zuhilfenahme von Heizoder Kühleinrichtungen, die für den nachfolgenden Überzug mit dem Beschichtungsmittel optimale Temperatur und Viskosität des Extrudats einstellen. In der Regel wird die Behandlung mit dem Beschichtungsmittel durchgeführt, wenn sich die Oberfläche des Extrudats gegenüber der Austrittstemperatur aus der Düse des Extruders um wenigstens 10 °C, bevorzugt um wenigstens 15 °C, abgekühlt hat oder abgekühlt wurde. Dadurch lassen sich auch mehrschichtige Dosierungsformen mit sehr unterschiedlichen Eigenschaften von Bindemittel und Beschichtungsmittel herstellen, beispielsweise mehrschichtige Dosierungsformen aus hochviskosen Bindemitteln oder Dosierungsformen mit sehr stark abweichenden Schmelz- oder Viskositätsverhalten von Innen- und Außenschichten.

Zur Herstellung von mehrschichtigen bzw. mehrfach beschichteten festen Dosierungsformen werden nach der ersten Behandlung mit einem Beschichtungsmittel ein oder mehrere weitere Beschichtungsmittel, die gleich oder verschieden sein können, aufgetragen. Vorzugsweise erfolgt die Behandlung mit mehreren Beschichtungsmitteln nacheinander, wobei der Abstand zwischen den Behandlungen in der Regel so gewählt wird, dass das (die) zuvor aufgetragene(n) Bindemittel zumindest teilweise erstarrt, getrocknet oder erhärtet ist (sind). In speziellen Ausführungsformen des erfindungsgemäßen Verfahrens wird das Extrudat mehrfach mit gleichen oder verschiedenen Beschichtungsmitteln behandelt, wobei mindestens eines der Beschichtungsmittel einen oder mehrere Wirkstoffe enthält.

Erfindungsgemäß verwendete Beschichtungsmittel sind vorzugsweise flüssige Beschichtungsmittel, wie Schmelzen, Lösungen, Dispersionen, Emulsionen oder Suspensionen. Jedoch sind auch dampfförmige Beschichtungsmittel geeignet, die durch Verdampfen von Flüssigkeiten und sublimierbaren Feststoffen erhältlich sind.

Als filmbildende Bestandteile der genannten flüssigen Beschichtungsmittel geeignet sind z. B. die zuvor bei den polymeren Bindemitteln genannten Polymere, insbesondere Polyvinylpyrrolidon (PVP), Copolymere aus N-Vinylpyrrolidon und Vinylestern, wie Vinylacetat.

Ebenfalls als filmbildende Bestandteile geeignet sind Gelatine, Polyvinylalkohol, Alkylcellulosen, wie Methylcellulosen oder Ethylcellulosen, Hydroxyalkylcellulosen, wie Hydroxyethyl-, Hydroxypropylcellulose oder Hydroxypropylmethylcellulose, Polyvinylpyrrolidon, bestimmte Acrylharze, wie z. B. Copolymerisate auf Basis von Dimethylaminoethylmethacrylaten und Methacrylestern (EudragitR-E), Polyvinylester, wie Polyvinylacetat, Polyalkylenglykole, wie Polypropylenglykole und Polyethylenglykole (z.B. Polyethylenglykol 6000), bestimmte Acrylharze, wie Copolymerisate auf Basis von Methacrylsäure und Methacrylsäureestern (EudragitR-L und -S), Cellulosephthalate, wie Celluloseacetatphthalat oder Hydroxpropylmethylcellulosephthalat und Mischungen davon. Bevorzugt enthält das Beschichtungsmittel als filmbildenden Bestandteil ein Cellulosederivat und/oder einen Polyvinylester.

Die Beschichtungsmittel können darüber hinaus Hilfsstoffe, wie Weichmacher, galenische Hilfsstoffe, Schmier- und Trennmittel, Fließmittel und ggf. Farbstoffe und/oder Wirkstoffe enthalten. Geeignete Substanzen sind z. B. bereits im Zusammenhang mit den polymeren Bindemitteln genannt.

Die filmbildenden Bestandteile können in dem Beschichtungsmittel, insbesondere bei der Behandlung des Extrudats mit dem Beschichtungsmittel, in geschmolzener, gelöster, dispergierter, suspendierter oder dampfförmiger Form vorliegen.

Die Beschichtungsmittel können Wasser oder mit Wasser mischbare Lösungsmittel, z. B. Ethanol, Isopropanol, Aceton, oder Gemische davon, enthalten. Die Menge an Lösungsmittel liegt im Allgemeinen im Bereich von 2 bis 30 Gew.-%, bezogen auf das Gesamtgewicht des Beschichtungsmittels.

Das Behandeln des Extrudats mit dem (den) Beschichtungsmittel(n) geschieht in der Regel kontinuierlich, vorzugsweise durch Tauchen, Gießen, Besprühen, Streichen oder Extrusionsbeschichten. Geeignete Vorrichtungen zum Auftragen von Beschichtungsmitteln sich dem Fachmann z. B. aus der Lacktechnologie und aus der Kunststoffverarbeitung (Ullmanns Enzyklopädie der chemischen Technik, Band XI, 3. Auflage, 1951, Seiten 367-376, Seiten 42-51, Seite 56-77) bekannt.

Vorteilhafterweise eignet sich das erfindungsgemäße Verfahren für die Herstellung von Beschichtungen sehr unterschiedlicher Dicken. In Abhängigkeit von der gewählten Form des Extrudats, der Art des Beschichtungsmittels und der Art der Behandlung des Extrudats mit dem Beschichtungsmittel können Gesamtschichtdicken zwischen 5 µm und 750 µm, vorzugsweise zwischen 10 µm und 500 µm, aufgebracht werden. Dabei werden wirkstofffreie Beschichtungsmittel oder Beschichtungsmittel mit geringem Wirkstoffgehalt (< 15 % Wirkstoff, bezogen auf das Beschichtungsmittelgesamtgewicht) vorzugsweise in einer Gesamtschichtdicke von 5 µm bis 250 µm und besonders bevorzugt von 10 µm bis 150 µm, aufgebracht. Bei stärker wirkstoffhaltigen Beschichtungsmitteln (Wirkstoffgehalt > 15 %, bezogen auf das Beschichtungsmittelgesamtgewicht) eignen sich insbesondere Gesamtschichtdicken von 150 µm bis 750 µm und bevorzugt 250 µm bis 500 µm.

Das Aufbringen der genannten Gesamtschichtdicken kann durch einfache oder mehrfache Behandlung mit dem (oder den) Beschichtungsmittel(n) erfolgen.

Zum Erreichen von bestimmten Schichtdicken oder Formen der Beschichtung wird das mit dem Beschichtungsmittel überzogene Extrudat vor dem Erhärten oder vollständigen Erhärten des Beschichtungsmittels durch eine kalibrierte Düse geführt.

In einer speziellen Ausführungsform des erfindungsgemäßen Verfahrens erfolgt das Behandeln des Extrudats durch Führen oder Ziehen des Extrudats durch eine Düse, wobei das Beschichtungsmittel durch einen Ringraum um die Düse zugeführt wird, und die Beschichtungsdicke durch anschließendes Abstreifen des Überschusses an der kalibrierten Düse eingestellt werden kann. Dabei kann die Zufuhr des Beschichtungsmittels sowohl in oder gegen Beschichtungsrichtung in sog. Axialspritzköpfen als auch schräg oder senkrecht zur Beschichtungsrichtung in sog. Schräg- oder Querspritzköpfen durchgeführt werden. Geeignete Spritzköpfe können auch eine Mehrfachbeschichtung in einem Arbeitsgang ermöglichen.

Nach der (oder den) Behandlung(en) erfolgt die Formung zu der gewünschten festen Dosierungsform. Dabei kann eine Vielzahl von Formen je nach Art der Formung erzeugt werden. Die Form kann z. B. über einen Heißabschlag, d. h. durch Zerschneiden bzw. Zerhacken des Stranges unmittelbar nach dem Austritt aus der Düse, oder über einen Kaltabschlag, d. h. durch Zerschneiden bzw. Zerhacken des Stranges nach zumindest teilweisem Abkühlen, separiert werden. Daran können sich weitere Formungsschritte anschließen. So können die durch Kaltabschlag oder insbesondere durch Heißabschlag erhaltenen Dosierungsformen mittels Arrondiervorrichtungen zur gewünschten Form arrondiert oder verrundet werden, wie in der DE-A 196 29 753 beschrieben.

Die Formung und/oder Vereinzelung zur gewünschten Dosierungsform kann vorteilhaft z. B. auch durch Bearbeiten des beschichteten Extrudats mittels einer Quetschvorrichtung, wie in der WO 97/15293 beschrieben, erfolgen. Gegebenenfalls können auch die mittels einer Quetschvorrichtung geformten festen Dosierungsformen in einem zweiten Schritt arrondiert werden.

Die Formung und/oder Vereinzelung zur gewünschten Dosierungsform kann vorteilhaft z. B. auch durch Bearbeiten des beschichteten Extrudats mittels eines Kalanders mit Formwalzen, wie in der EP-A 240 904, EP-A 240 906, EP-A 358 105 und WO 96/19962 beschrieben, erfolgen. Die Form der Vertiefungen und damit der geformten Dosierungsformen kann weitgehend beliebig gewählt werden.

Mit den genannten Verfahren lassen sich vorteilhaft eine Vielzahl von geformten festen Dosierungsformen herstellen, z. B. Pellets, runde Tabletten, Oblong-Tabletten, teilbare Tabletten, linsenförmige Tabletten, Dragees und Zäpfchen.

## Patentansprüche

1. Verfahren zur Herstellung von beschichteten festen Dosierungsformen durch Bilden eines plastischen Gemischs, enthaltend mindestens ein thermoplastisches physiologisch verträgliches polymeres Bindemittel und mindestens einen Wirkstoff, und Extrudieren des plastischen Gemischs, wobei man anschließend das Extrudat mit mindestens einem flüssigen oder dampfförmigen Beschichtungsmittel behandelt und das beschichtete Extrudat zur gewünschten Dosierungsform formt, **dadurch gekennzeichnet, dass** man das mit dem Beschichtungsmittel überzogene Extrudat zur Einstellung der Beschichtungsdicke und der Beschichtungsform durch eine kalibrierte Düse führt.

2. Verfahren nach Anspruch 1, **dadurch gekennzeichnet, dass** das Überziehen des Extrudats mit dem Beschichtungsmittel durch Tauchen in das oder Besprühen mit dem Beschichtungsmittel erfolgt.

3. Verfahren nach Anspruch 2, **dadurch gekennzeichnet, dass** das Beschichtungsmittel als Schmelze, Lösung, Dispersion, Emulsion oder Suspension eingesetzt wird.

4. Verfahren nach einem der vorhergehenden Ansprüche, **dadurch gekennzeichnet, dass** die Dosierungsform aus dem beschichteten Extrudat durch Heißabschlag, Kaltabschlag, Abquetschen oder Kalandrieren mittels Formwalzen geformt wird.

5. Verfahren nach einem der vorhergehenden Ansprüche, **dadurch gekennzeichnet, dass** mindestens eines der eingesetzten Beschichtungsmittel einen oder mehrere Wirkstoffe enthält.

6. Verfahren nach einem der vorhergehenden Ansprüche, **dadurch gekennzeichnet, dass** das Beschichtungsmittel als filmbildenden Bestandteil ein Cellulosederivat und/oder einen Polyvinylester enthält.

7. Verfahren nach einem der vorhergehenden Ansprüche, **dadurch gekennzeichnet, dass** das thermoplastische Bindemittel ausgewählt ist unter Polyvinylpyrrolidon, Copolymerisaten von N-Vinyllactamen und Vinylestern, Copolymerisaten von N-Vinyllactamen und (Meth)acrylsäureestern, Polyhydroxyalkylacrylaten, Polyhydroxyalkylmethacrylaten, Polyacrylaten, Polymethacrylaten, Alkylcellulosen und Hydroxyalkylcellulosen.

8. Verfahren nach einem der vorhergehenden Ansprüche, **dadurch gekennzeichnet, dass** das thermoplastische Bindemittel einen K-Wert nach Fikentscher von > 45 aufweist.

9. Verfahren nach einem der vorhergehenden Ansprüche, **dadurch gekennzeichnet, dass** es sich bei dem zu beschichtenden Extrudat um ein zwei- oder mehrschichtiges Coextrudat handelt.

## Claims

1. A process for producing coated solid dosage forms by forming a plastic mixture comprising at least one thermoplastic physiologically tolerated polymeric binder and at least one active ingredient and extruding the plastic mixture, wherein the extrudate is subsequently treated with at least one liquid or vaporized coating agent, and the coated extrudate is shaped to the required dosage form, wherein the extrudate coated with the coating agent is passed through a calibrated die to adjust the coating thickness and the coating shape.

2. A process as claimed in claim 1, wherein the coating of the extrudate with the coating agent takes place by dipping into or spraying with the coating agent.

3. 'A process as claimed in claim 2, wherein the coating agent is employed as melt, solution, dispersion, emulsion or suspension.

4. A process as claimed in any of the preceding claims, wherein the dosage form is shaped from the coated extrudate by a hot cut, cold cut, pinching off or calendering using molding rolls.

5. A process as claimed in any of the preceding claims, wherein at least one of the coating agents employed comprises one or more active ingredients.

6. A process as claimed in any of the preceding claims, wherein the coating agent comprises as film-forming ingredient a cellulose derivative and/or a polyvinyl ester.

7. A process as claimed in any of the preceding claims, wherein the thermoplastic binder is selected from polyvinylpyrrolidone, copolymers of N-vinyllactams and vinyl esters, copolymers of N-vinyllactams and (meth)acrylic esters, poly(hydroxyalkyl acrylates), poly(hydroxyalkyl methacrylates), polyacrylates, polymethacrylates, alkylcelluloses and hydroxyalkylcelluloses.

8. A process as claimed in any of the preceding claims, wherein the thermoplastic binder has a Fikentscher K value > 45.

9. A process as claimed in any of the preceding claims, wherein the extrudate to be coated is a two-layer or multilayer coextrudate.

## Revendications

1. Procédé pour la préparation de formes galéniques solides enrobées, par formation d'un mélange plastique, contenant au moins un liant polymère physiologiquement acceptable, thermoplastique, et au moins une substance active, et extrusion du mélange plastique, tandis qu'on traite ensuite l'extrudat avec au moins un agent de revêtement liquide ou sous forme gazeuse et on confectionne l'extrudat revêtu pour lui conférer la forme galénique souhaitée, **caractérisé par le fait qu'**on fait passer l'extrudat revêtu avec l'agent de revêtement à travers une buse calibrée pour ajuster la densité du revêtement et la forme du revêtement.

2. Procédé selon la revendication 1, **caractérisé par le fait qu'**on effectue le revêtement de l'extrudat avec l'agent d'enrobage par immersion dans ou aspersion avec l'agent d'enrobage.

3. Procédé selon la revendication 2, **caractérisé par le fait que** l'agent d'enrobage est utilisé sous forme de masse fondue, de solution, de dispersion, d'émulsion ou de suspension.

4. Procédé selon l'une des revendications précédentes, **caractérisé par le fait que** la forme galénique est mise en forme à partir d'un extrudat enrobé par coupe à chaud, coupe à froid, écrasement ou calandrage au moyen de rouleaux de profilage.

5. Procédé selon l'une des revendications précédentes, **caractérisé par le fait qu'**au moins l'un des agents de revêtement utilisés contient une ou plusieurs substances actives.

6. Procédé selon l'une des revendications précédentes, **caractérisé par le fait que** l'agent de revêtement contient comme composant filmogène un dérivé de cellulose et/ou un poly(ester vinylique).

7. Procédé selon l'une des revendications précédentes, **caractérisé par le fait que** le liant thermoplastique est choisi parmi la poly(pyrrolidone de vinyle), des copolymères de N-vinyllactames et d'ester de vinyle, des copolymères de N-vinyllactames et d'esters d'acide (méth)acrylique, des acrylates de polyhydroxyalkyle, des méthacrylates de polyhydroxyalkyle, des alkylcelluloses et des hydroxyalkylcelluloses.

8. Procédé selon l'une des revendications précédentes, **caractérisé par le fait que** le liant thermoplastique présente un indice K selon Fikentscher > 45.

9. Procédé selon l'une des revendications précédentes, **caractérisé par le fait qu'**il s'agit, pour l'extrudat à revêtir, d'un coextrudat à deux ou plusieurs couches.
